Europäisches Patentamt

European Patent Office

Office européen des brevets

(19)

(11) Veröffentlichungsnummer: **0 442 008 A1**

## (12) EUROPÄISCHE PATENTANMELDUNG

(21) Anmeldenummer: **90102927.2**

(22) Anmeldetag: **15.02.90**

(51) Int. Cl.5: **A61M 16/00**

(43) Veröffentlichungstag der Anmeldung:
**21.08.91 Patentblatt 91/34**

(84) Benannte Vertragsstaaten:
**AT BE CH DE DK ES FR GB GR IT LI LU NL SE**

(71) Anmelder: **MAHRT UND HOERNING GMBH**
**Am Handweisergraben 13**
**W-3406 Bovenden(DE)**

(72) Erfinder: **Siemsen, Bernhard**
**Hopfengarten 10**
**W-3406 Bovenden(DE)**

(54) **Beatmungstubus für die Notfallbeatmung.**

(57) Zur Überwindung der Ekelschwelle bei der Mund-zu-Mund-Beatmung Bewußtloser und zur Vermeidung gegenseitiger Infizierung mit ansteckenden Krankheiten besteht der Tubus aus einem gebogenen gegen Durchbeißen verstärkten Ansatz (A), die die Zunge des Verletzten hindert, zurückzufallen und die Luftröhre zu verschließen. Eine Abdeckplatte (B) schließt den Mund des Atemempfängers ab und verhindert zugleich unmittelbaren Mund-zu Mund-Kontakt. Auf der Spenderseite gestattet ein Normkonus (C) das Mundstück (D) oder Beatmungsbeutel in beliebiger Stellung zu adaptieren.

In das Mundstück (D) ist ein hydrophober Filter (F) eingeschweißt, der bei ungehinderter Durchlässigkeit der Atemluft während der Mund-zu-Mund-Beatmung Blut und Speichel beidseitig zurückhält.

EP 0 442 008 A1

## BEATMUNGSTUBUS FÜR DIE NOTFALLBEATMUNG

Die Erfindung bezieht sich auf einen Tubus, der für die Notfallbeatmung von Bewußtlosen konzipiert worden ist.

Bewußtlose, z.B. infolge von Unfällen, sind häufig nur durch sofortige Mund- zu- Mund- Beatmung zu retten, Obgleich die Technik dieser Notfallbeatmung in den Erste-Hilfe-Kursen vermittelt wird, scheitert ihre Anwendung häufig zum einen an der sog. Ekelschwelle, die Hilfswillige nicht zu überwinden vermögen, zum anderen an der Furcht, sich durch aus dem Mund blutende Verletzte oder Kontakt mit deren Speichel zu infizieren (Aids, Hepatitis, TB ). Die gleiche Ansteckungsgefahr besteht für den Verletzten durch mit der Atemluft des Spenders übertragenen Speichel oder Blut.

Versucht wird, dem mit verschiedenartigen Beatmungshilfen zu begegnen. So werden Tücher und Masken verwendet, die die Mundpartie des Bewußtlosen abdecken und den unmittelbaren Mund- zu Mund-Kontakt verhindern. Ein Nachteil dieser Hilfsmittel liegt darin, daß mit ihnen nicht vermieden werden kann, daß die Zunge des Bewußtlosen während des Beatmungsvorganges zurückfällt und die Luftröhre verschließt. Überdies wird der nahezu ungehinderte Austausch von Blut und Speichel und die damit verbundene Anstekkungsgefahr nicht beseitigt. Bekannte Tuben mögen in der Lage sein, die Notfall-Beatmung ohne Erstickungsgefahr für den Verletzten zu gewährleisten. Es sind auch Tuben mit Einweg-Ventilen (Lippen-Ventilen) bekannt, die jedoch nur einseitig verhindern, daß Blut und Speichel des Verletzten in den Mund des Spenders gelangen können. Eine Ansteckungsgefahr für den Spender ist damit nicht beseitigt. Aus der Patentschrift FR-A-160919 ist die Verwendung eines Tubus mit kurzem Ansatzstück, trichterförmiger Abdeckung des Empfängermundes und trichterförmigem Mundstück auf der Spenderseite bekannt. Eine ununterbrochene Weiterbeatmung durch Beatmungsgeräte und eine sichere Verhinderung des Feuchtigkeitsaustausches zwischen Spender und Empfänger ist nicht möglich.

Der Erfindung liegt die Aufgabe zugrunde, die Ekelschwelle durch Vermeidung unmittelbaren Mund- zu- Mund-Kontakt zu überwinden, die Zunge des Bewußtlosen während des Beatmungsvorganges zurück- und damit die Luftröhre frei zu halten, zu verhindern, daß Atemluft des Spenders nutzlos entweicht, daß während der Beatmungsdauer ohne Beeinträchtigung der Durchlässigkeit Blut und Speichel in beiden Richtungen gleichermaßen sicher zurückgehalten werden und daß mit Beatmungssystemen des Rettungswesens ohne Wechsel des Tubus die Notfall-Beatmung ohne Unterbrechung fortgesetzt werden kann.

Dieses Ziel wird erfindungsgemäß durch einen zweiteiligen Notfall-Beatmungs-Tubus erreicht, dessen gebogener Ansatz in den Mund des zu Beatmenden geschoben wird, und mit einem adaptierbaren Mundstück, dadurch gekennzeichnet, daß der Ansatz einen 15 mm-Normkonus auf der Spenderseite für den Anschluß handelsüblicher Beatmungsbeutel oder des Mundstücks aufweist, und daß das Mundstück auf der spenderentfernten Seite einen 15 mm- Normkonus aufweist, der in jeder beliebigen Stellung auf den Tubus aufgesteckt werden kann, beide Teile hergestellt aus einem thermoplastischen Kautschuk-Kunststoff.

Der Ansatz ist mit einer anatomisch geformten Abdeckplatte versehen, die den Mund des Atemempfängers verschließt und zugleich verhindert, daß der Ansatz durch zu tiefes Hineinschieben in der Mund des zu Beatmenden diesen verletzt. Auf den Normkonus des Spenderseite des Ansatzstücks können Rettungssysteme oder das Mundstück des Tubus adaptiert werden, in das ein hydrophober Filter in Ultraschweiß-Technik fest eingearbeitet ist, der beidseitig Blut und Speichel für eine Dauer von mindestens 5 Minuten bei einem Atemdruck p von 5 Millibar und einer Luftfeuchte von 100 % sicher zurückhält.

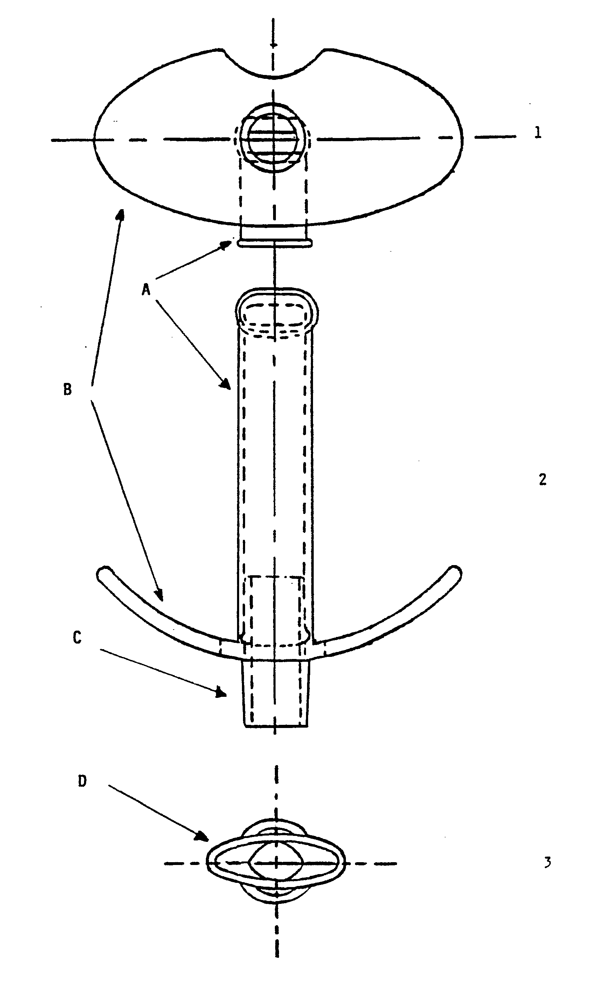

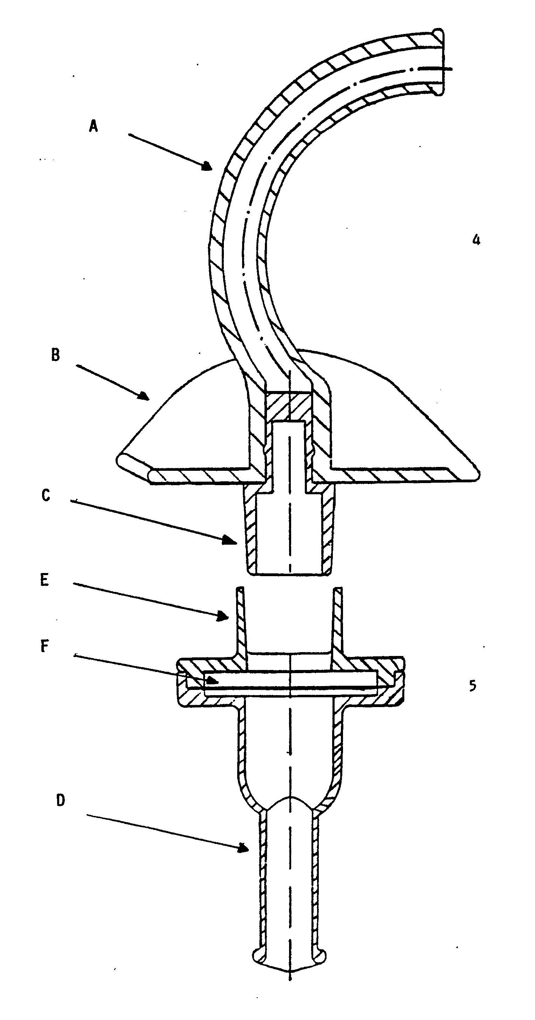

Ein Ausführungsbeispiel mit seinen verschiedenen Teilen ist im Querschnitt (Zeichnungen 1, 2 und 3) und Längsschnitt (Zeichnungen 6 und 5) dargestellt. Der Tubus besteht aus einem gebogenen Ansatz (A) der in den Mund des Bewußtlosen geschoben wird, sowie aus einem adaptierbaren Mundstück (Zeichnung 3 und 5 ). Er verfügt über eine anatomisch geformte Abdeckplatte für den Abschluß des Tubus am Mund des Verletzten (B). Der Ansatz weist auf der Spenderseite einen 15 mm-Normkonus (C) auf für den Anschluß des Mundstückes oder handelsüblicher Beatmungsbeutel. Das Mundstück ist auf der Spenderseite oval geformt (D); es kann in jeder beliebigen Stellung auf den Konus (C) geschoben werden. Der gebogene Ansatz des Tubus ist im distalen Bereich abgerundet, um Verletzungen in der Mundhöhle des Atemempfängers zu vermeiden. Er ist im proximalen Bereich durch den Normkonus (C) verstärkt, um ein Durchbeißen des Tubus durch den Atemempfänger zu verhindern. Das Mundstück enthält außerdem einen in Ultraschweiß-Technik eingearbeiteten hydrophoben Filter (E).

**Patentansprüche**

1. Notfall-Beatmungs-Tubus mit einem gebogenen Ansatz (A), der in den Mund des zu Beatmenden geschoben wird, und mit einem adaptierbaren Mundstück (D), dadurch gekennzeichnet, daß der Ansatz einen 15 mm-Normkonus auf der Spenderseite (C) für den Anschluß handelsüblicher Beatmungsbeutel oder des Mundstücks aufweist, daß das Mundstück auf der spenderentfernten Seite einen 15 mm-Normkonus (E) aufweist, der in jeder beliebigen Stellung auf den Tubus aufgesteckt werden kann, beide Teile hergestellt aus einem thermoplatischen Kautschuk-Kunststoff.

2. Notfall-Beatmungs-Tubus nach Anspruch 1, gekennzeichnet durch eine anatomisch geformte Abdeckplatte (B) für den Anschluß des Tubus am Mund des zu Beatmenden.

3. Notfall-Beatmungs-Tubus nach Anspruch 1, gekennzeichnet dadurch, daß das Mundstück (D) auf der Spenderseite oval geformt ist.

4. Notfall-Beatmungs-Tubus nach Anspruch 1, gekennzeichnet dadurch, daß der Ansatz (A) im distalen Bereich abgerundet ist.

5. Notfall-Beatmungs-Tubus nach Anspruch 1, gekennzeichnet dadurch, daß der Ansatz im proximalen Bereich durch den Normkonus (C) verstärkt ist.

6. Notfall-Beatmungs-Tubus nach Anspruch 1, gekennzeichnet dadurch, daß in das Mundstück (D) ein hydrophober Filter (F) mit einem Durchmesser von 28 mm fest eingeschweißt ist, der bei ungehinderter Durchlässigkeit der Atemluft beidseitig Blut und Speichel für eine Dauer von mindestens 5 Minuten bei einem Atemdruck von $\triangle$ p von fünf Millibar und einer Luftfeuchte von 100 % zurückhält.

4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | WO-A-8 904 189 (MAHRT & HOERNING)<br>* Ganzes Dokument *<br>----- | 1-6 | A 61 M 16/00 |

RECHERCHIERTE SACHGEBIETE (Int. Cl.5)

A 61 M

Der vorliegende Recherchenbericht wurde für alle Patentansprüche erstellt

| Recherchenort | Abschlußdatum der Recherche | Prüfer |
|---|---|---|
| DEN HAAG | 12-10-1990 | JONES T.M. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
..................................................................................................
& : Mitglied der gleichen Patentfamilie, übereinstimmendes Dokument

EPO FORM 1503 03.82 (P0403)